Europäisches Patentamt

European Patent Office

Office européen des brevets

(11) Veröffentlichungsnummer: **0 300 298**
**A2**

## EUROPÄISCHE PATENTANMELDUNG

(21) Anmeldenummer: 88110995.3

(22) Anmeldetag: 09.07.88

(51) Int. Cl.⁴: **C12N 15/00**

(30) Priorität: 24.07.87 DE 3724482

(43) Veröffentlichungstag der Anmeldung:
25.01.89 Patentblatt 89/04

(84) Benannte Vertragsstaaten:
AT BE CH DE ES FR GB GR IT LI LU NL SE

(71) Anmelder: BOEHRINGER MANNHEIM GMBH
Sandhofer Strasse 116
D-6800 Mannheim 31(DE)

(72) Erfinder: Schaffner, Walter, Prof. Dr.
Bodenstrasse 9
CH-8104 Weiningen(CH)
Erfinder: Gerster, Thomas, Dr. phil.
500 E 63rd Street Apt. 5G
New York, N.Y. 10021(US)

(54) Verfahren zur Erweiterung der Zellspezifität von eukaryotischen Expressionsvektoren.

(57) Ein Verfahren zur Erweiterung der durch ein erstes Enhancer-Sequenzmotiv vorgegebenen Zelltyp-Spezifität eines eukaryotischen Expressionsvektors, welches dadurch gekennzeichnet ist, daß in den Vektor mindestens ein weiteres identisches Enhancer-Sequenzmotiv im Abstand von 10 - 100 Basenpaaren vom ersten Motiv eingebaut wird. Damit ist eine Expression von Proteinen in anderen Zellen als nur in den durch das erste Enhancer-Sequenzmotiv vorgegebenen Zellen sowie in Zellen, für die bisher noch kein Expressionssystem bekannt war, möglich.

Fig.2

```
CTGTGGTTTG AAGAAGTGGT TTTGAAACAC TCTGTCCAGC CCCACCAAAC

CGAAAGTCCA GGCTGAGCAA AACACCACCT GGGTAATTTG CATTTCTAAA

ATAAGTTGAG GATT
```

EP 0 300 298 A2

## Verfahren zur Erweiterung der Zellspezifität von eukaryotischen Expressionsvektoren

Die Erfindung betrifft ein Verfahren zur Erweiterung der Zellspezifität von eukaryotischen Expressionsvektoren und ihre Verwendung zur Expression von Proteinen in Säugerzellen.

Die Expression von Proteinen in Säugerzellen mit eukaryotischen Vektoren ist aus pharmazeutischer und diagnostischer Sicht von besonderem Interesse.

Die Expression von Proteinen in Säugerzellen mit eukaryotischen Vektoren ist bisher nur für wenige Zellinien (z.B. CHO-Zellen, DNA 3 (1984) 297- 308, Mol. and Cell. Biol. 5 (1985) 1750 - 1759 und Mol. and Cell. Biol. 4 (1984) 166 - 172, Adeno-Vektor in Hela-Zellen, [P.N.A.S. 82 (1985). 3567 - 3571) bekannt und verläuft dabei, im Vergleich zur Expression in Prokaryonten, nur mit schwacher Ausbeute.

Üblicherweise werden in Säugerzellen Vektoren mit Elementen von Viren verwendet. Beispiele hierfür sind Bovine papilloma virus [P.N.A.S. 81 (1984 5086 - 5090], Simian virus 40 [P.N.A.S. 82 (1985). 3644 - 3648], retroviral elements [DNA 4 (1985) 23-31] oder adenovirus elements [P.N.A.S. 82 (1985). 3567 - 3571].

Solche Vektoren basieren meist auf eukaryotischen Virus-DNA-Strukturen und enthalten im wesentlichen das Gen des zu exprimierenden Proteins, falls erforderlich Resistenzgene für die Selektion. einen Promotor, einen Ursprungspunkt der DNA-Replikation (ori) sowie aktivierende DNA-Sequenzen (Enhancer-Elemente) (vgl. Science 209 (1980), 1422 - 1427). Für die Präparation von Vektor-DNA in Bakterien kann der Vektor in einem bakteriellen Plasmid kloniert sein. Die Enhancer-Elemente sind meist oberhalb (5´) des zu exprimierenden Gens, entweder außerhalb oder übergehend in die Promotor-Sequenz. angeordnet. Enhancer-Elemente entfalten ihre Aktivität auch in einer Position innerhalb oder auch unterhalb (3´) der Transkriptionseinheit. Die Länge dieser Elemente beträgt meist zwischen 70 und 300 bp (SV40 Enhancer. Polyomavirus Enhancer. Nucl. Acids Res. 10 (1982). 7965 - 7976), aber es wurden auch schon wesentlich längere Enhancer-Elemente gefunden (Cell 41 (1985). 521 - 530. P.N.A.S. 82 (1985) p. 8325 - 8329). Die Enhancer-Elemente können die eukaryotische Transkription stimulieren und sind deshalb von besonderem Interesse. Diese stimulierende Wirkung ist weitgehend unabhängig von der Lage und Orientierung des Enhancer-Elements im Vektor (Cell. 27 (1981), 299 - 308).

Enhancer-Elemente werden in eukaryotischen Viren (z.B., SV40, Polyomavirus. BPV. Cytomegalovirus. MSV. HIV) sowie in eukaryotischen Genen. wie z.B. den Genen für Immunglobulin. Interferon. Chymotryp sin und Insulin. gefunden. Bei SV40 ist die Enhancer-Funktion größtenteils in einem 72 bp langen Repeat, welcher sich oberhalb (5´) vom Transkriptionsstart befindet. lokalisiert (Cell. 27 (1981), 299 - 308). In diesem 72 bp Repeat ist eine core-Sequenz enthalten, die wesentlich zur Enhancer-Funktion beiträgt (Science 219 - (1983), 626 - 631). Eine solche core-Sequenz ist jedoch allein kein brauchbarer Enhancer (Genes Dev. 1 - (1987) 65 - 73). Sie muß in beträchtlichem Umfang von ihren natürlichen Nachbarsequenzen umgeben sein (Cell. 39 (1984), 653 - 662). Aber auch mit derartigen Enhancer-Elementen konnte bisher nur eine Aktivität von maximal 5 % des Wildtyps erreicht werden (Mol. and Cell. Biol. 5 (1985), 649 - 658). Eine wesentliche Erhöhung der Expression findet statt, wenn die Enhancer-Elemente multimerisiert werden. So konnte für den Polyomavirus-Enhancer mit einem Segment von 26 Nucleotiden. welches in 5 - 7 Kopien im Vektor enthalten war. sogar eine geringfügig höhere Transkriptionsrate als beim Wildtyp beobachtet werden (Mol. and Cell. Biol. 5 (1985) . 649 - 658).

Ein Charakteristikum der meisten dieser Vektorsysteme ist außerdem. daß sie streng zellspezifisch sind.

IgH-Enhancer sind beispielsweise nur in lymphoiden Zellen aktiv [Cell 33, 729 - 740 (1983)]. Die Expression des Insulin-Gens in den endokrinen Langerhans-Zellen wird von einem Zelltyp-spezifischen Enhancer gesteuert (Cell 45, (1986) 35 - 44, Nature 315, (1985) 115 - 122). Zudem ist bekannt. daß die Expres sion des Elastase-Gens in den exokrinen Pankreas-Zellen von einem Zelltyp-spezifischen Enhancer Promotor-Element gesteuert wird (Nature 313, (1985), 600 - 602).

Für die Herstellung von therapeutischen Säugerproteinen wäre dagegen anzustreben. daß bei der gentechnologischen Herstellung tatsächlich authentisches Material hergestellt werden kann. Dies bedeutet. daß die Expression in dem Zelltyp erfolgen sollte. in dem dieses Protein auch in der Natur exprimiert wird.

Für die therapeutische Anwendung am Menschen sollte also tPA in humanen Endothelzellen.α-Interferon in humanen Leukozyten, β-Interferon in humanen Fibroblasten und Erythropoetin in humanen Nierenzellen exprimiert werden. Damit könnte die naturidentische Glycosylierung (z.B. bei tPA) die γ-Carboxylierung (z.B. Faktor VIII) die Phosphorylierung (z.B. Kasein) sowie die korrekte Sulfatierung (z.B. Proteoglykane) erreicht werden.

Außerdem sind für viele Zelltypen geeignete Enhancer überhaupt nicht bekannt. wodurch die effektive Expression von Fremdproteinen bisher auf nur wenige Eukaryonten-Zelltypen beschränkt ist.

Aufgabe der vorliegenden Erfindung ist deshalb. ein Verfahren zur Verfügung zu stellen. mit dem eine Erweiterung der Zelltyp-Spezifität von eukaryotischen Expressionsvektoren erreicht werden kann.

Diese Aufgabe wird durch ein Verfahren zur Erweiterung der durch ein erstes Enhancer-Sequenzmotiv vorgegebenen Zelltyp-Spezifität eines eukaryotischen Expressionsvektors gelöst, welches dadurch gekennzeichnet ist, daß in den Vektor mindestens ein weiteres identisches Enhancer-Sequenzmotiv im Abstand von 10 - 100 Basenpaaren (bp) vom ersten Motiv eingebaut wird.

Überraschenderweise hat sich gezeigt, daß mit dem erfindungsgemäßen Verfahren die Expression von Vektoren, deren Zelltyp-Spezifität durch ein Enhancer-Sequenzmotiv geprägt ist, erweitert werden kann. Beispielsweise gelingt es mit dem erfindungsgemäßen Verfahren, für Vektoren, die ein Enhancer-Sequenzmotiv des humanen IgH-Enhancers enthalten, außer der Spezifität für B-Zellen, also Zellen des hämopoetischen Systems, zusätzliche Spezifität für Bindegewebszellen, beispielsweise für LMTK⁻, NIH3T3 und/oder 3T6-Zellen zu erreichen.

Unter Enhancer-Sequenzmotiven sind diejenigen Sequenzen eines Enhancers zu verstehen, die für die Enhancer-Wirkung essentiell sind und demzufolge als der funktionell wirksame Bereich des Enhancers bezeichnet werden können. Die Länge eines dieser Enhancer-Sequenzmotive beträgt üblicherweise zwischen 6 und 18 bp. Diese Sequenzmotive zeigen häufig eine Homologie mit den Enhancern aus anderen Viren und zellulären Genen. Beispiele für solche Sequenzmotive sind:

$^A_C$ GGAAGTGA$^A_C$ (Ela core enhancer, Cell 33, 695 - 703 (1983))

GTGG$^A_T$ $^A_T$ $^A_T$ G (Sequenz homolog zu Immunoglobulin und viralen Enhancern, Science 219, 626 - 631 (1983)

TGTGGTAAG (MSV, PNAS 79 (1982) 6453)

GTGTGGAAAG (Science 219, 626 - 631 (1983))

TGGTTTG (BPV, Cell 18 (1979) 963)

GTGTGGTTT (Py(F101), Nucleic Acids Res. 9 (1981) 6251).

ATGCAAATNA (Nature 310 (1986), 71-74)

TGAGTCAG (AP-1 Motiv, Cell 49 (1987) 729 - 739, 741 - 752)

$^G_3$ GGGCGG$^G_A$ $^G_A$ $^C_T$ (Bindungsstelle für Transkriptionsfaktor SP1, Trends in Biochem. Sci. 11 (1986), 20 - 23)

TNATTTGCAT (IgH enhancer, Cell 33 (1983) 729 - 740)

TCAAGATGGC, AGCAGCTGGC, GTCATGTGGC, ACCACCGGGT (Ephrussi-Motive des Maus IgH-Enhancers, Science 227 (1983) 134 - 140).

Besonders vorteilhaft ist es, Enhancer-Sequenzmotive von, zellulären Genen (z.B. des menschlichen ncers, Nature 306, 806 - 809 (1983) zu verwenden. Damit kann die Zellkultur frei von viralen Elementen gehalten werden.

Als Enhancer-Sequenzmotive sind auch solche Sequenzen geeignet, die eine weitgehende Homologie mit den genannten Enhancer-Sequenzmotiven aufweisen. Eine weitgehende Homologie im Sinne der Erfindung ist dann noch gegeben, wenn bis zu 2 Nukleotide des Sequenzmotivs gegen beliebige andere Nukleotide ausgetauscht sind.

Die Anzahl der zusätzlich eingebauten Enhancer-Sequenzmotive beträgt 1 - 12, vorzugsweise 3 - 10, und besonders bevorzugt 3 - 8 Enhancer-Sequenzmotive.

Sofern die maximale Expression des heterologen Proteins erreicht werden soll, können die Enhancer-Sequenzmotive durch Nachbarsequenzen, welche die Sequenzmotive natürlicherweise auf beiden Seiten flankieren, zu Enhancersubelementen erweitert werden. Sofern jedoch nicht die maximale Expression gewünscht wird, können, unter Erhalt der Zellspezifität, Punktmutationen in die Nachbarsequenzen eingefügt werden. Dies führt jedoch zu einer Verringerung der Genexpression [Science 219 (1983) 626 - 631]. Derselbe Effekt ist auch durch eine geringe Kopienzahl von Enhancer Subelementen bzw. Enhancer-Sequenzmotiven zu erreichen (Genes Dev. 1 (1987) 65 - 73).

Die Enhancer-Subelemente haben vorteilhaft eine Länge von 15 - 120 bp und enthalten 1 - 8, vorzugsweise 1 - 4 Enhancer-Sequenzmotive.

Besonders geeignete Enhancer-Subelemente sind

TGAGCAAAACACCACCTGGGTAATTTGCATTTCTAAAATAAGTTGAGGATT

(Segment des IgH-Enhancers, 51 bp), und

ATCTTAGAAATTGTTGAACTAGTTCCAAACATCCTGTGGTTGCAATGTAG

(Segment der H. saimiri-Sv40 recombinanten Virus DNA, EMBO 4 (1985), 2669 - 2674). Für das H. saimiri-Segment ist das enthaltene Enhancer-Sequenzmotiv nicht lokalisiert worden.

Die Enhancer-Sequenzmotive bzw. Enhancer-Subelemente sind 20 - 2000 bp oberhalb (5′) vom Transkriptionsstart des zu exprimierenden Gens oder 20 - 2000 bp unterhalb (3′) des zu exprimierenden Gens orientierungsunabhängig eingebaut.

Der bevorzugte Abstand ist bei -30 bis -50 bp oberhalb (5′) der TATA-BOX oder bei Verwendung eines kompletten Promotors bis etwa -110 bp oberhalb des Promotors und/oder bis ca. -1500 bp oberhalb der ganzen Transkriptionseinheit. In einer weiteren Ausführungsform wird das erfindungsgemäße DNA-Segment unterhalb (3′) der ganzen Transkriptionseinheit eingebaut. Die Enhancer-Sequenzmotive bzw. Enhancer-Subelemente sind im Vektor im Abstand von 10 - 100 bp eingebaut. Die Zwischensequenzen entsprechen vorzugsweise Sequenzen, die in natürlichen Enhancern nicht vorkommen. Besonders bevorzugt werden Multilinker-Sequenzen als Zwischensequenzen verwendet.

Unter einem Multi-Linker ist eine DNA-Sequenz zu verstehen, welche einer Aneinanderreihung von erkennungssequenzen für bakterielle Restriktionsenzyme entspricht. Die Restriktionserkennungssequenzen sind meist als inverted repeats organisiert. Vorzugsweise wird ein symmetrischer Multilinker verwendet. Bevorzugt eingesetzt werden folgende Multilinker:

GACGGATCCGGGGAATTCCCCGGATCCGTC

(Abwandlung aus pUC7)

GAATTCGAGCTCGGTACCCGGGGATCCTCTAGAGTCGACCTGCAGGCATGCAAGCTT

(Abwandlung aus pUC18)

Multilinker des Vektors Bluescript M13 (Strategene, San Diego, USA)

Sofern der Multilinker geeignete terminale Sequenzen hat, kann er direkt mit den Enhancer-Subelementen bzw. den Enhancer-Sequenzmotiven ligiert werden. In den anderen Fällen müssen zunächst kompatible Enden für die Ligierung erzeugt werden. Dies geschieht durch Auffüllen von vorstehenden Enden mit Klenow Polymerase oder T4 Polymerase. Vorzugsweise werden flache Enden (blunt ends) für die Ligation verwendet.

Vorzugsweise werden Multilinker verwendet die gleichlang sind. Es ist auch bevorzugt, daß die Sequenzen so ausgewählt sind, daß sie unter den oben genannten Standardbedingungen miteinander hybridisieren. Besonders bevorzugt ist es, palindromische Multilinker zu verwenden. Hierbei wird nur jeweils eine Hälfte der Sequenz zur Hybridisierung verwendet.

Die Länge des Multilinkers kann 10 - 100 bp bevorzugt 20 - 60 bp sein.

Ein weiterer Gegenstand der Erfindung ist die Verwendung eines eukaryotischen Expressionsvektors, welcher mindestens zwei identische, zelltypspezifische Enhancer-Sequenzmotive im Abstand von 10 - 100 bp enthält, zur Expression in Zellen anderen Zelltyps.

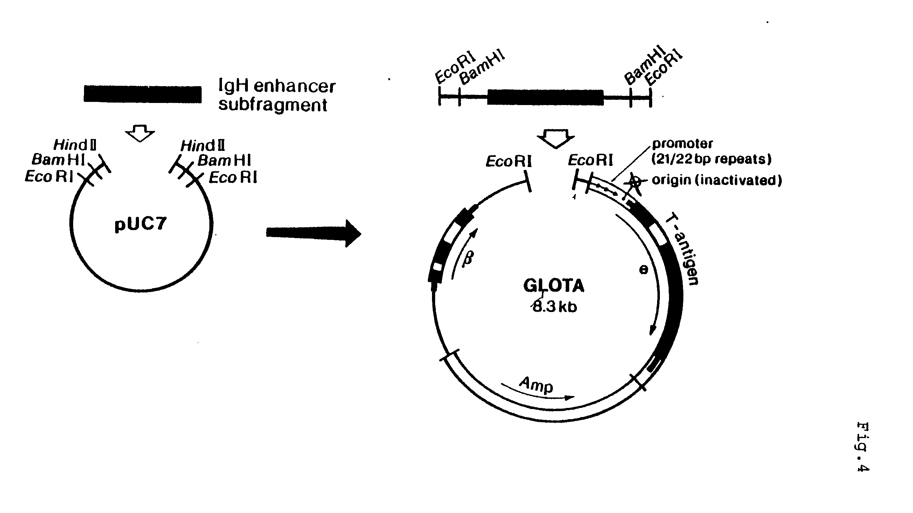

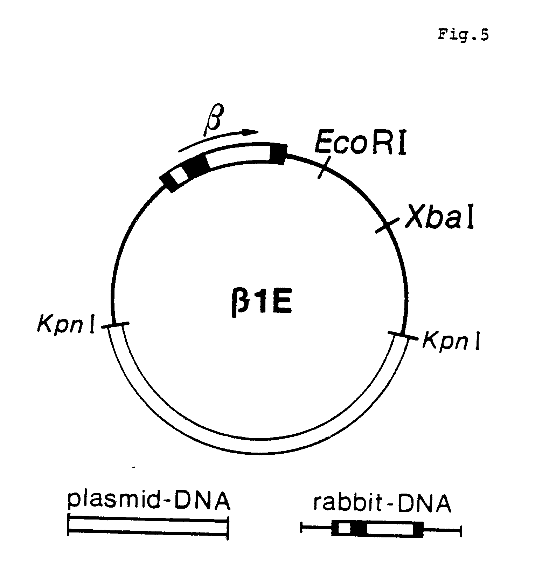

Die Vektoren können nach den dem Fachmann geläufigen gentechnologischen Standardverfahren hergestellt werden. Geeignete Ausgangsvektoren sind z.B. pβG (Cell 33 (1983), 729 - 740)), G10TA (Fig. 4. DSM 4096P) und pβ1E (Fig. 5, DSM 4097P) sowie deren Derivate (Cell 41 (1985) 521 - 530). Diese Vektoren zeichnen sich dadurch aus, daß sie Elemente für effiziente Transkription (Enhancer, Promotor) und Processing Sequenzen (z.B. großes Intron des Globin--Gens) Sequenzen für Polyadenylierung (z.B. AATAAA Motiv) und eine Terminatorsequenz für die Transkription (Cell. 40 (1985) 897- 907) enthalten. Es hat sich auch gezeigt, daß das ganze Globin Gen in seiner unveränderten Form geeignet ist. Abkömmlinge davon zur Expression eines heterologen Gens sind z.B. der in EMBO J.5 (1986) 1921 - 1972 beschriebene Vektor. Der weitere Aufbau der Vektoren ergibt sich wie weiter oben ausgeführt.

Die nachfolgenden Beispiele und Abbildungen erläutern die Erfindung weiter.

In den Abbildungen stellen dar:

Fig. 1 Sequenz des 51 bp-IgH-Enhancer-Subelements

Fig. 2 Sequenz des 114 bp-IgH-Enhancer-Subelements

Fig. 3 Sequenz des (symmetrischen) 15 bp Multilinkers

Fig. 4 Konstruktion von Vektoren mit IgH-Enhancer-Subelementen

Fig. 5 Plasmid β1E

Die gentechnologischen Standardverfahren, wie Klonierung, DNA-Isolierung, Restriktion, werden, wenn nichts anderes angegeben ist, analog T. Maniatis et al., Molecular Cloning (1982) Cold Spring Harbor, New York, 11724 durchgeführt. Die molekularbiologischen Reagenzien werden entsprechend den Angaben der Hersteller eingesetzt.

Beispiel 1:

**Herstellung von Vektoren mit 51 bp-IgH-Enhancer-Subelementen**

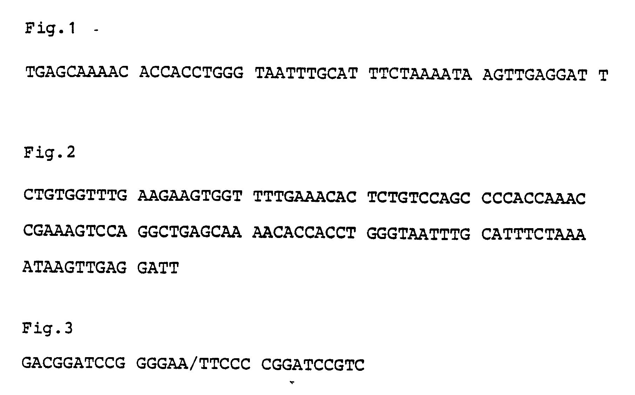

Ein Restriktionsfragment, welches einen Teil der IgH-Enhancer-Sequenz enthält (Gerster, EMBO J., 6 - (1987) 1323 - 1330) (Fig. 1), wird aufgefüllt und in die HindII-Stellen des symmetrischen Polylinkers von pUC7, Gene 19 (1982), 259 - 268, eingesetzt. Es werden Rekombinanten isoliert, welche die Inserts als Monomere enthalten. Bei der Behandlung mit Eco RI entstehen Fragmente, welche ein IgH-Enhancer-Fragment enthalten, welches auf beiden Seiten mit je 15 bp Polylinker-DNA (Sequenz Fig. 3) flankiert ist.

Diese Fragmente werden bei hoher Konzentration in die single Eco RI site des Vektors GLOTA (DSM 4096P) ligiert, der das Kaninchen-$\beta$1-Globin-Gen und das Sv40-T-Antigen enthält (Fig. 4).

Es werden folgende Plasmide isoliert:

GB31: 3 x 51 bp IgH-Sequenz (E) mit jeweils 30 bp Polylinker (P) zwischen den IgH-Sequenzen (E-P-E-P-E)

GB51: 5 x 51 bp IgH-Sequenz (E) mit jeweils 30 bp Polylinker(P) zwischen den IgH-Sequenzen (E-P-E-P-E-P-E-P-E)

Beispiel 2:

## Herstellung von Vektoren mit 114 bp-IgH-Enhancer-Subelementen

Ein Restriktionsfragment, welches einem 114 bp Fragment des IgH-Enhancers entspricht (Sequenz Fig. 2), wird, wie in Beispiel 1 beschrieben, mit aufgefüllten Enden in die HindII Stelle von pUC7 und dann weiter in die EcoRI Stelle von GLOTA kloniert.

Insgesamt werden folgende Plasmide isoliert:

GC31: 3 x 114 bp IgH-Sequenz (E) ohne Polylinker zwischen den IgH-Sequenzen (E-E-E)

GC33: 3 x 114 bp IgH-Sequenz (E) mit jeweils 30 bp Polylinker (P) zwischen den IgH-Sequenzen (E-P-E-P-E)

Beispiel 3:

## Transfektion

.Zu 1 µg DNA in 600 µl TBS (tris buffered saline, 25 mmol/l Tris pH 7,4, 137 mmol/l NaCl, 5 mM KCl und 0,6 mM Dinatriumhydrogensulfat)) werden 500 µg DEAE-Dextran gegeben. Anschließend werden Zellen, welche mit TBS gewaschen wurden, zugegeben und eine halbe Stunde inkubiert. Die Mischung wird mit 3 ml 25% (v/v) DMSO für 3 min behandelt. Anschließend wird 2mal mit TBS gewaschen.

Es wird ein Dulbecco-modifiziertes Eagle-Medium verwendet, welches für XS63 -Zellen 10 % (v/v) FKS (fötales Kälberserum) enthält. Für die anderen Zellen wird anstelle 10 % FKS nur 2,5 % FKS mit 2,5 % Serum von neugeborenen Kälbern zugesetzt.

Das Zellwachstum nach der Transfektion erfolgt in 36 - 42 Stunden in Petrischalen.

Beispiel: 4:

## Bestimmung der gebildeten mRNA (mittels Sp6 Kartierungsmethode)

36 - 42 Stunden nach Transfektion werden die Zellen geerntet. Hierzu wird das Medium abgesaugt, einmal mit TBS gewaschen und mit Trypsin-EDTA behandelt. Nichthaftende Zellen wie XS63 müssen dazu durch Zentrifugation pelletiert werden. Anschließend werden die Zellen in 10 ml Medium resuspendiert, wieder gewaschen und zentrifugiert.

Das Präzipitat wird lysiert und nach Zentrifugation der Überstand mit dem Zytoplasma weiter verarbeitet. 320 µl des Überstandes werden mit 40 µl 10 (w/v) %igem SDS und 20 µl Proteinase K behandelt. Nach einer Inkubation von 30 min bei 37 - 60° C wird mit Phenol/Trichlormethan (1:1) behandelt und nach Zentrifugation der Überstand mit 25 µl 3 mol/l Natriumacetat, pH 7, und 2,5 Volumina Ethanol versetzt.

Der Niederschlag wird in 178 µl $H_2O$ resuspendiert. Anschließend werden 20 µl zehnmal DNase-Puffer zugesetzt. Nach Zusatz von weiteren 2 µl RNase-freier DNase (1 U/µl) wird bei 37° C für 30 min inkubiert. Anschließend wird auf pH 7 mit 0,3 mol/l Natriumacetatpuffer eingestellt und 2mal mit Phenol/Dichlormethan extrahiert. Anschließend wird mit Äthanol präzipitiert.

Anschließend wird ein Sp6-mapping (Melton, Nucl. Acids Res. 12, (1984) 7035 - 7056) durchgeführt. Hierzu wird als DNA-Probe das 902 bp BglII/BamHI Fragment des $\beta$1-Globin Gens in SP64 Vektor verwendet, welches mit einem Teil der $\beta$-Globin-mRNA hybridisiert.

Die Hybridisierung wird bei 37°C mit einem Hybridisierungspuffer (80 % (v/v) Formamid, 40 mmol/l Pipes pH 6.4, 400 mmol/l Natriumchlorid, 1 mmol/l EDTA) durchgeführt.

Anschließend werden 300 μl 0,3 mol/l Natriumacetatpuffer pH 7.0, 5 mmol/l EDTA, 4 - 8 μg/ml RNase A und 8 - 16 U/ml RNase T1 zugegeben. Nach einer Inkubation von 1 Stunde bei 30 - 37°C wird mit 2.5 μl Proteinase K und 7 μl 10 %igem SDS für 15 min bei 37 - 50°C behandelt.

Anschließend wird mit Phenol Dichlormethan extrahiert und mit Äthanol präzipitiert. Das Präzipitat wird in Formamid blue juice resuspendiert, 3 min bei 90°C erhitzt und auf ein 8 mol/l Harnstoff-PAGE-Gel gegeben. Die Ergebnisse sind in Tabelle 1 zu sehen.

Tabelle 1:  Aktivität von Expressionsvektoren in
verschiedenen Zellen
(Angaben in Prozent der Aktivität des
SV40 Enhancers)

| Expressions- vektoren | Zellinien[2] | | | |
| --- | --- | --- | --- | --- |
| | ATCC TIB17 XS63 | ATCC CCL1.3 L-M(TK⁻) | ATCC CRL1658 NIH/3T3 | ATCC CCL36 3T6-Swiss albino |
| kompletter IgH enhancer | 70 | 0 | 0 | 0 |
| GB31 | 80 | 20 | _[1] | _[1] |
| GB51 | 100 | 30 | 150 | 120 |
| GC31 | 30 | _[1] | 0 | 0 |
| GC33 | 70 | 10 | 0 | 30 |

[1] nicht gemessen

[2] Die Zellinien sind unter den genannten ATCC-Nummern bei der American Type Culture Collection 12301 Parklawn Drive, Rockville, MD 20852, USA, erhältlich.

**Ansprüche**

1. Verfahren zur Erweiterung der durch ein erstes Enhancer-Sequenzmotiv vorgegebenen Zelltyp-Spezifität eines eukaryotischen Expressionsvektors, dadurch gekennzeichnet, daß in den Vektor mindestens ein weiteres identisches Enhancer-Sequenzmotiv im Abstand von 10 - 100 Basenpaaren (bp) vom ersten Motiv eingebaut wird.

2. Verfahren nach Anspruch 1, dadurch gekennzeichnet, daß in den Vektor 1 - 12 weitere identische Enhancer-Sequenzmotive jeweils im Abstand von 10 - 100 bp vom ersten Motiv eingebaut werden.

3. Verfahren nach Anspruch 1 oder 2, dadurch gekennzeichnet, daß als Enhancer-Sequenzmotiv $^A_C$ GGAAGTG$^A_C$ , GTGG$^A_T$ $^A_T$ $^A_T$ G, TGTGGTAAG, GTGTGGAAAG, TGGTTTG, GTGTGGTTT, ATGCAAATNA, TGAGTCAG, $^G_A$ GGGCGG$^G_A$ $^G_A$ $^C_T$ , TNATTTGCAT, TCAAGATGGC, AGCAGCTGGC, GTCATGTGGC oder ACCACCGGGT verwendet wird.

4. Verfahren nach Anspruch 1 - 3, dadurch gekennzeichnet, daß die Enhancer-Sequenzmotive in Enhancer-Subelemente mit einer Länge von 15 - 120 bp eingebunden werden.

5. Verfahren nach Anspruch 1 - 4, dadurch gekennzeichnet, daß als Enhancer Subelement TGAGCAAAACACCACCTGGGTAATTTGCATTTCTAAAATAAGTTGAGGATT
oder
ATCTTAGAAATTGTTGAACTAGTTCCAAACATCCTGTGGTTGCAATGTAG
verwendet wird.

6. Verfahren nach Anspruch 1 - 5, dadurch gekennzeichnet, daß die Enhancer-Sequenzmotive oder die Enhancer-Subelemente durch Multilinker verbunden werden.

7. Verfahren nach Anspruch 1 - 6, dadurch gekennzeichnet, daß die Enhancer-Sequenzmotive oder Enhancer-Subelemente 20 - 2000 bp oberhalb (5´) vom Transkriptionsstart des zu exprimierenden Gens oder 20 - 2000 bp unterhalb (3´) des zu exprimierenden Gens orientierungsunabhängig eingebaut werden.

8. Verwendung eines eukaryotischen Expressionsvektors mit einer durch ein erstes Enhancer--Sequenzmotiv vorgegebenen Zelltyp-Spezifität, welcher mindestens ein weiteres identisches Enhancer-Sequenzmotiv im Abstand von 10 - 100 bp vom ersten Motiv eingebaut enthält zur Expression in Zellen anderer Zelltyp-Spezifität.

9. Verwendung eines eukaryotischen Expressionsvektors nach Anspruch 8, welcher 1 - 12 weitere identische Enhancer-Sequenzmotive jeweils im Abstand von 10 - 100 bp vom ersten Motiv eingebaut enthält.

10. Verwendung eines eukaryotischen Expressionsvektors nach den Ansprüchen 8 und 9, welcher als Enhancer-Sequenzmotiv $^A_C$ GGAAGTG$^A_C$ , GTGG$^A_T$ $^A_T$ $^A_T$ G, TGTGGTAAG, GTGTGGAAAG, TGGTTTG, GTGTGGTTT, ATGCAAATNA, TGAGTCAG, $^G_A$ GGGCGG$^G_A$ $^G_A$ $^C_T$ , TNATTTGCAT, TCAAGATGGC, AG-CAGCTGGC, GTCATGTGGC oder ACCACCGGGT enthält.

11. Verwendung eines eukaryotischen Expressionsvektors nach den Ansprüchen 8 - 10, welcher die Enhancer-Sequenzmotive in Enhancer-Subelemente mit einer Länge von 15 - 120 bp eingebunden enthält.

12. Verwendung eines eukaryotischen Expressionsvektores nach Anspruch 10, dadurch gekennzeichnet, daß dieser als Enhancer-Subelement
TGAGCAAAACACCACCTGGGTAATTTGCATTTCTAAAATAAGTTGAGGATT
oder
ATCTTAGAAATTGTTGAACTAGTTCCAAACATCCTGTGGTTGCAATGTAG enthält.

13. Verwendung eines eukaryotischen Expressionsvektors nach den Ansprüchen 8 - 12, welcher die Enhancer-Sequenzmotive oder die Enhancer-Subelemente durch Multilinker verbunden enthält.

14. Verwendung eines eukaryotischen Expressionsvektors nach den Ansprüchen 8 - 13, welcher die Enhancer-Sequenzmotive oder Enhancer-Subelemente 20 - 2000 bp oberhalb (5´) vom Transkriptionsstart des zu exprimierenden Gens oder 20 - 2000 bp unterhalb (3´) des zu exprimierenden Gens orientierungsu-nabhängig eingebaut enthält.

Fig.1 -

TGAGCAAAAC ACCACCTGGG TAATTTGCAT TTCTAAAATA AGTTGAGGAT T

Fig.2

CTGTGGTTTG AAGAAGTGGT TTTGAAACAC TCTGTCCAGC CCCACCAAAC

CGAAAGTCCA GGCTGAGCAA AACACCACCT GGGTAATTTG CATTTCTAAA

ATAAGTTGAG GATT

Fig.3

GACGGATCCG GGGAA/TTCCC CGGATCCGTC

IgH enhancer subfragment

pUC7

*Hind* II
*Bam* HI
*Eco* RI

*Hind* II
*Bam* HI
*Eco* RI

*Eco*RI
*Bam*HI

*Bam*HI
*Eco*RI

*Eco*RI

*Eco*RI

promoter
(21/22 bp repeats)

origin (inactivated)

T-antigen

β

e

GLOTA
8.3kb

Amp

EP 0 300 298 A2

Fig. 4

Fig.5